# EUROPEAN PATENT SPECIFICATION

(11) **EP 1 165 135 B1**
(45) Date of publication and mention of the grant of the patent: **01.09.2004**
(21) Application number: 00918345.0
(22) Date of filing: 24.03.2000
(51) Int. Cl.: A61K 45/00, C12Q 1/68, C12Q 1/60, C12Q 1/26, A61P 9/10, C07K 14/705, G01N 33/74

(54) **METHODS OF SCREENING FOR COMPOUNDS MODULATING FXR RECEPTORS**
VERFAHREN ZUR SELEKTION VON FXR-REZEPTORMODULATOREN
PROCEDES DE CRIBLAGE DE COMPOSES MODULANT L'ACTIVITE DES RECEPTEURS FXR

(30) Priority: 26.03.1999 US 126334 P
(43) Date of publication of application: 02.01.2002
(60) Divisional application: 04075173.7
(73) Proprietor: City of Hope, Duarte California 91010 (US)
(72) Inventor: FORMAN, Barry, M., Newport Beach, CA 92660 (US); WANG, Haibo, Madera, CA 93637 (US)
(74) Representative: Marchant, James Ian
(86) International application number: PCT/US2000/007836
(87) International publication number: WO 2000/057915

(56) References cited:
- WO-A-94/18346
- WO-A-97/35195
- US-A- 5 736 157
- FORMAN BARRY M ET AL: "Identification of a Nuclear Receptor That Is Activated by Farnesol Metabolites." CELL, vol. 81, no. 5, June 1995 (1995-06), pages 687-693, XP000919168 ISSN: 0092-8674
- WEINBERGER CARY: "A model for farnesoid feedback control in the mevalonate pathway." TRENDS IN ENDOCRINOLOGY AND METABOLISM, vol. 7, no. 1, 1996, pages 1-6, XP000916741 ISSN: 1043-2760

## Description

### BACKGROUND

Cholesterol is essential for a variety of cellular activities, including membrane biogenesis, steroid and bile acid biosynthesis, caveolae formation and covalent protein modification. The widespread utilization of cholesterol in different metabolic pathways signifies that minimal blood concentrations must be maintained. On the other hand, an excess of circulating cholesterol is a major risk factor in the development of atherosclerotic heart disease, the single largest cause of mortality in the United States which accounts for nearly 500,000 deaths each year.

Circulating cholesterol levels are regulated by cellular uptake, synthesis and degradation (Brown and Goldstein, Cell, 89:331-340 (1997)). Removal of excess cholesterol is complicated by the fact that it is an insoluble lipid, most of which is embedded within cell membranes. The major route for cholesterol degradation is metabolic conversion to bile acids, which are less hydrophobic and hence more easily removed from the cell than cholesterol. The conversion to bile acids occurs exclusively in the liver. One chemical pathway for this conversion is initiated by cholesterol 7α-hydroxylase (Cyp7a), the rate-limiting enzyme in this pathway. In humans, cholesterol is converted to bile acids by both the 7α-hydroxylase and the sterol 27-hydroxylase pathways.

In vivo, bile acids are metabolized by hepatocytes and intestinal microorganisms, producing a large number of different products. The existence of so many chemically related products and complex biochemical pathways make the isolation and study of the effects of individual components difficult. See Elliott and Hyde, Am. J. Med., 51:568-579 (1971). For example, chenodeoxycholic acid (CDCA; 5β-cholanic acid-3α, 7α-diol) and cholic acid (CA; 5β-cholanic acid-3α, 7α, 12α-triol) are two of the major end products of bile acid biosynthesis (Chiang, Front. Biosci., 3:D176-193 (1998); Vlahcevic et al., Hepatology, 13:590-600 (1991)). Both are produced exclusively in the liver where they can be further metabolized by conjugation with taurine or glycine. These bile acids are secreted into the intestine, reabsorbed in the ileum, and transported back to the liver via the portal circulation. During their transit in the intestine, primary bile acids such as CDCA and CA undergo microbial mediated 7α-dehydroxylation and are converted to lithocholic acid (LCA; 5β-cholanic acid-3α-ol) and deoxycholic acid (DCA; 5β-cholanic acid-3α, 12α-diol), respectively (Elliott and Hyde, Am. J. Med., 51:568-579 (1971); Hylemon, "Metabolism of Bile Acids in Intestinal Microflora" in Sterols and Bile Acids, H. Danielsson and J. Sjovall, eds. (New York, Elsevier), pp. 331-344 (1985)).

In addition to their metabolic functions, bile acids also act as signaling molecules that negatively regulate their own biosynthesis. In particular, biliary components act in a negative feedback loop that limits bile acid production by inhibiting expression of the Cyp7a enzyme. While it is known that several bile acid components can induce this negative feedback regulation, the nature of the bile acid sensor which transduces the bile acid signal and the mechanism by which it does so have heretofore remained unknown. Inhibition of Cyp7a is known to occur at the transcriptional level, however, and negative bile acid response elements have been found in the Cyp7a promoter. Bile acids also have been shown to down-regulate sterol 27-hydroxylase, the enzyme involved in conversion of cholesterol to bile acids through a different pathway. See Twisk et al., Biochem. J., 305:505-521 (1995).

Nuclear receptors are ligand-modulated transcription factors that mediate the transcriptional effects of steroid, thyroid and retinoid hormones. These receptors have conserved DNA-binding domains (DBD) which specifically bind to the DNA at cis-acting elements in the promoters of their target genes and ligand binding domains (LBD) which allow for specific activation of the receptor by a particular hormone or other factor. Transcriptional activation of the target gene for a nuclear receptor occurs when the circulating ligand binds to the LBD and induces a conformation change in the receptor that facilitates recruitment of a coactivator. Coactivator recruitment results in a receptor complex which has a high affinity for a specific DNA region and which can modulate the transcription of the specific gene. Recruitment of a coactivator after agonist binding allows the receptor to activate transcription. Binding of a receptor antagonist induces a different conformational change in the receptor such that coactivator recruitment results in non-productive interaction with the basal transcriptional machinery of the target gene. As will be apparent to those skilled in the art, an agonist of a receptor that effects negative transcriptional control over a particular gene will actually decrease expression of the gene. Conversely, an antagonist of such a receptor will increase expression of the gene.

At least two classes of nuclear receptor coactivators have been identified. The first class includes the CBP and SRC-1 related proteins that modulate chromatin structure by virtue of their histone acetylase activity. A second class includes PBD/DRIP 205/TRAP 220 which is part of a large transcriptional complex that is postulated to interact directly with the basic transcriptional machinery.

In addition to the known classical nuclear hormone receptors that respond to specific, identified hormones, several orphan receptors have been identified which lack known ligands. These orphan receptors include, for example, FXR, CARβ, PPARα, PPARδ, TR2-11, LXRα, GCNF, SF1, RORα, Nurr1, DAX and ERR2. The orphan receptor FXR (farnesoid X-activated receptor) is known to inhibit Cyp7a. It binds to its response element as a heterodimer with RXR (9-cis retinoic acid receptor) which can be activated by RXR ligands.

It has been hypothesized that orphan receptors act as sensors for some metabolic signals, including fatty acids, prostanoids and metabolites of farnesol and cholesterol. Ever since the pioneering studies on the lac operon, it has been well established that intermediary metabolites serve as signaling molecules in bacteria and yeast (Gancedo, Microbiol. Mo. Biol. Rev., 62:334-361 (1998); Ullmann, Biochimie, 67:29-34 (1985)). Understanding of metabolite control in mammalian cells has been hampered by the need to identify metabolic signals and their cognate sensors.

Previous studies on the bile acid-signaling pathway in liver were performed using intact animals or cultured hepatocytes. Workers using studies of this design were not able to discover which compounds were the ultimate bile acid signaling molecules because the bile acids were subject to metabolic conversion in these systems by intestinal microorganisms and/or liver-specific enzymes into a number of different products. The receptor which acts as a sensor for cholesterol and bile acid signals thus had not been identified.

Because inhibition of cholesterol catabolism by bile acids limits the amount of cholesterol that can be excreted as bile acids, identification of the nuclear receptor which regulates bile acid and cholesterol metabolism would be a major advantage in developing treatment modalities for individuals with hypercholesterolemia or other conditions related to activation levels of a gene regulated by this receptor. The current inability to interfere with the transcription or expression of bile acid regulated genes, for example the negative feedback imposed by bile acids on cholesterol catabolism, poses a serious stumbling block in treating individuals with conditions related to these genes, for example hypercholesterolemia. Any condition involving a defect in the regulation of a bile acid nuclear receptor controlled gene would be ameliorated by modification of the receptor activity. The nuclear bile acid sensor has been shown to respond to bile acids by either stimulating or suppressing target gene transcription. These activities are mediated by positive FXR response elements within these genes. For example, bile acids coordinately repress the transcription of the liver- and ileal/renal-specific bile acid transporters. (Torchia et al., Biochem. Biophys. Res. Commun. 225:128-133 (1996), sterol 27-hydroxylase (Cyp27) (Twisk et al., Biochem. J., 305:505-511 (1995) and sterol 12α-hydroxylase (Cyp12) (Einarsson et al., J. Lipid Res., 33:1591-1595 (1992)). Therefore, detrimental metabolic conditions which could be ameliorated by either stimulation or suppression of a bile acid receptor target gene may be treated with bile acid receptor ligands.

There is consequently a need for compounds and methods to modulate the expression of genes regulated by bile acids. A further need exists for a screening method for identifying compounds that can provide a pharmacologic intervention to modify the regulation of transcription of bile acid regulated genes. Such compounds and methods would be of value to patients who would benefit from modification of bile acid regulated gene transcription, such as individuals suffering from hypercholesterolemia.

### SUMMARY OF THE INVENTION

The present invention provides a method of screening for compounds useful in modulating FXR-mediated gene transcription which comprises contacting a mixture of FXR and RXR with a compound and determining whether said compound promotes formation of an FXR-RXR heterodimer, wherein the RXR is an RXR mutant (RXRm) which contains a functional DNA-binding domain and which has a mutation in the ligand-binding domain and which prevents substantial activation by RXR ligands but which does not otherwise substantially affect the ability of the RXR mutant receptor to form heterodimers with FXR.

The invention further provides a method of screening compounds for FXR antagonist activity which comprises contacting a mixture of FXR, RXR and a known FXR agonist with a compound and determining whether said compound inhibits the agonist-promoted formation of an FXR-RXR heterodimer, wherein the RXR is an RXR mutant (RXRm) which contains a functional DNA-binding domain and which prevents substantial activation by ligands but which does not otherwise substantially affect the ability of the RXR mutant receptor to form heterodimers with FXR. One such method for screening compounds for cholesterol catabolism-modulating activity comprises (1) providing a first mixture which contains (i) an FXR receptor, (ii) an RXR receptor, and (iii) a labelled DNA probe which contains a sequence to which the DNA-binding domain of a ligand-FXR-RXR complex specifically binds, (2) providing a second mixture which contains (i) an FXR receptor, (ii) an RXR mutant receptor ("RXRm") which contains a functional DNA-binding domain and which has a mutation in the ligand-binding domain which prevents activation by RXR ligands but does not otherwise substantially affect the ability of the RXR mutant receptor to form heterodimers with FXR or of such heterodimers to recruit coactivator, (iii) a labelled DNA probe which contains a sequence to which the DNA-binding domain of a ligand-FXR-RXR complex specifically binds, (3) contacting said first and second mixtures with the compound being screened, (4) determining whether the compound being screened causes binding of an FRX-RXR heterodimer to the DNA probe, and (5) determining whether the compound being screened causes binding of an FXR-RXRm heterodimer to the DNA probe. In another embodiment of the screening method, the method further comprises contacting the first and second mixtures with a known FXR ligand and selecting compounds that inhibit the ability of said known FXR ligand to cause the FXR-RXR heterodimer to bind to the DNA probe. The above methods may be used to screen for compounds having FXR antagonist activities by including in the first mixture and the second mixture a known FXR ligand and determining in steps (4) and (5) whether the compound being screened inhibits binding of the heterodimers to the DNA probe.
Additionally, methods in which the coactivator is a polyeptide or active fragment thereof which contains a peptide motif that interacts with the FXR-RXR heterodimer in a ligand-dependent manner may be used with this invention.

Another embodiment of the invention provides a method for screening for compounds useful in modulating FXR-mediated gene transcription which comprises contacting a mixture of FXR, RXR and an FXR/RXR coactivator with a compound and determining whether said compound promotes coactivator recruitment by an FXR-RXR heterodimer, wherein the RXR is an RXR mutant ("RXRm") which contains a functional DNA-binding domain and which has a mutation in the ligand-binding domain which prevents substantial activation by RXR ligands but which does not otherwise substantially affect the ability of the RXR mutant receptor to form heterodimers with FXR or of such heterodimers to recruit coactivator.

Another further embodiment of the invention provides a method of screening compounds for FXR antagonist activity which comprises contacting a mixture of FXR, RXR, an FXR/RXR coactivator and a known FXR agonist with a compound and determining whether said compound inhibits the agonist promoted coactivity recruitment by an FXR-RXR heterodimer, wherein the RXR is an RXR mutant ("RXRm") which contains a functional DNA-binding domain and which has a mutation in the ligand-binding domain which prevents substantial activation by RXR ligands but which does not otherwise substantially affect the ability of the RXR mutant receptor to form heterodimers with FXR or of such heterodimers to recruit coactivator.

Yet another screening method embodiment of the invention is a cellular system which comprises: (a) transfecting mammalian cells with a gene encoding FXR under control of an operative promoter; (b) transfecting said cells with an operative reporter gene under control of a promoter linked to a DNA sequence which encodes an operative response element to which ligand-activated FXR or FXR complex binds to initiate transcription of said reporter gene; (c) culturing said cells in the presence of a compound being screened; and (d) monitoring said cells for transcription or expression of the reporter gene as an indication of FXR activation, wherein the cells are transfected with a gene encoding a bile acid transporter molecule under control of an operative promoter. When conducted in the presence of a known FXR ligand, the method is useful for identifying FXR antagonists. Expression of the transporter molecule effects transport of hydrophilic molecules across the cell membrane, thus enabling detection of their ability to modulate activation of the FXR receptor.

Other embodiments of the invention, in accordance with the scope of the claims, will be appreciated by those skilled in the art.

### BRIEF DESCRIPTION OF THE DRAWINGS

Figure 1 provides data from an activation assay of FXR-RXR heterodimers and several other orphan receptors with bile extract. The receptors, each of which is known in the art and is further identified herein by its Genbank accession number are CARβ, PPARα, PPARδ, TR2-11, LXRα, GCNF, SF1, RORα, Nurr1, DAX, and ERR2.
Figure 2 provides data comparing the activation by a synthetic specific RXR ligand, LG268, of chimeric receptors containing the yeast GAL4 DNA binding domain fused to the wild-type RXR ligand binding domain or a mutant RXR (RXRm) ligand binding domain.
Figure 3 is an autoradiogram showing results of electrophoretic mobility experiments demonstrating the formation of receptor-coactivator complexes with wild-type RXR (RXR-CoA) or mutant RXR (RXRm-CoA) at increasing concentrations of the RXR ligand, LG268.
Figure 4 provides quantitative data for the results shown in Figure 3.
Figure 5 provides data comparing activation of RXR and FXR heterodimers by the RXR ligand LG268 and a bile extract.
Figure 6 shows FXR, RXR and RXRm activation data of fractions obtained from preparative thin layer chromatography of bile extract.
Figure 7 shows the reversed phase HPLC absorbance tracing of fraction B from Figure 6.
Figure 8 provides data showing that HPLC peak Z from Figure 7 potently activated FXR-RXRm but has no effect on RXR.
Figure 9 shows the level of FXR activation by several different free bile acids. Juvenile hormone III and the RXR ligand LG268 were included as controls. UDCA indicates ursodeoxycholic acid (5β-cholanic acid-3α,7β-diol).
Figure 10 shows the level of FXR activation by CA, CDCA, DCA and LCA, unconjugated or conjugated with either glycine or taurine, in the presence or absence of a liver bile acid transporter.
Figure 11 provides FXR activation does-response data for CDCA, DCA and LCA. The EC50 for each of the compounds was approximately 50 µM.
Figure 12 summarizes structure-activity information derived from the data given in the previous Figures. ++ indicates >200-fold activation and + indicates 100-150-fold activation of FXR-RXR heterodimers.
Figure 13 provides data showing that CDCA and LCA both activate transcription in cells co-expressing a GAL-L-FXR chimera and the RXR LBD (L-RXR).
Figure 14 shows data demonstrating activation after recruitment of a GAL-4 coactivator fusion protein (GAL-CoA) which was dependent on the presence of both the RXR and FXR LBDs in a mammalian two-hybrid assay.
Figure 15 shows electrophoretic mobility data demonstrating coactivator recruitment by different ligands in the presence of FXR and RXR or FXR and RXRm.
Figure 16 shows the activation of an LXR reporter gene by LXR, RXR or both in the presence of FXR (left panel) and the activation of the T3R (Triiodothyronine receptor) by T3Rβ, RXR or both in the presence of FXR and added ligand (right panel).

### DETAILED DESCRIPTION OF THE INVENTION

This invention provides a method for identifying compounds which activate or inhibit FXR.

It has been found that extracts of bile specifically activate the orphan receptor FXR. An active endogenous bile acid signaling molecule which activates this receptor was purified to homogeneity and identified as chenodeoxycholic acid (CDCA). Further analysis and structure-activity studies revealed that CA, DCA and LCA also activate FXR. Additionally, LCA was found to promote coactivator recruitment *in vitro.*

To verify that a bile acid component was able to bind to and activate the FXR orphan receptor, an extract of porcine bile (Sigma) was prepared and tested on a number of orphan nuclear receptors. The receptors to be tested were expressed in CV-1 cells, which are derived from COS cells. The cells are described in Boyer et al., Am. J. Physiol, 266:G382-G387 (1994).

Use of a standard model heterologous cell system to reconstitute bile acid responsiveness allows activity to be monitored in the absence of the metabolic events which may obscure the process being tested. Any suitable heterologous cell system may be used to test the activation of potential or known bile acid nuclear receptor ligands, as long as the cells are capable of being transiently transfected with the appropriate DNA which expresses receptors, reporter genes, response elements, and the like. Cells which constitutively express one or more of the necessary genes may be used as well. Cell systems that are suitable for the transient expression of mammalian genes and which are amenable to maintenance in culture are well known to those skilled in the art.

**Table I.**

| Reporter/Receptor Pairs for Orphan Receptor Activation Assay | | |
|---|---|---|
| No. | Reporter | Receptor(s) |
| 1 | EcRE x 6 | FXR + RXR |
| 2 | βRE2 x 3 | CARβ |
| 3 | PPRE x 3 | PPARα,δ, TR2-11 |
| 4 | LXRE x 3 | LXRα |
| 5 | DR0 x 2 | GCNF |
| 6 | SF1 x 4 | SF1 |
| 7 | UAS_{G} x 4 | GAL-RORα, GAL-Nurr1, GAL-DAX, GAL-ERR2 |

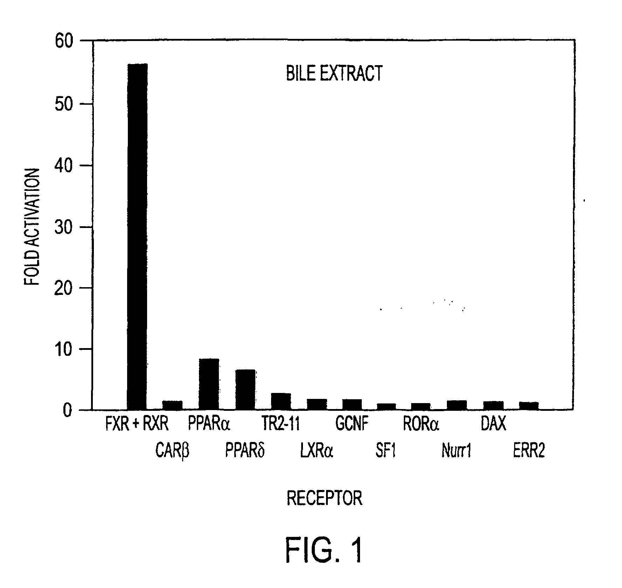

To test the activation of various orphan receptors by bile acids, CV-1 cells were transiently transfected with expression vectors for the receptors indicated in Figure 1 along with appropriate reporter constructs according to methods known in the art. Suitable reporter gene constructs are well known to skilled workers in the fields of biochemistry and molecular biology. The reporter/receptor pairs used in the assay reported in Figure 1 are listed in Table I. All transfections additionally contained CMX-βgal as an internal control. Suitable constructs for use in the these studies may conveniently be cloned into pCMX. pCMX contains the cytomegalovirus promoter/enhancer followed by a bacteriophage T7 promoter for transcription in vitro. Other vectors known in the art car be used in the methods of the present invention.

Genes encoding the following full-length previously described proteins, which are suitable for use in the studies described herein, were cloned into pCMX: rat FXR (accession U18374), human RXRα (accession X52773), human TRβ (accession X04707, human LXRα (accession U22662), mouse PPARα (accession X57638), mouse PPARδ (accession U10375), human TR2-11 (accession M29960), mouse GCNF (accession u14666), mouse SF1 (accession S65878). All accession numbers in this application refer to GenBank accession numbers. GAL4 fusions containing receptor fragments were constructed by fusing the following protein sequences to the C-terminal end of the yeast GAL4 DNA binding domain (amino acids 1-147) from pSG424 (Sadowski and Ptashne, Nucl. Acids Res., 17:7539 (1989)): GAL-L-RXR (human RXRα Glu 203 - Thr 462), GAL-L-FXR (rat FXR LBD Leu 181 - Gln 469), GAL-RORα (human RORα1 Arg 140 - Gly 523, accession U04897), GAL-Nurr1 (mouse Nurr1, Cys 318 - Phe 598, accession S53744), GAL-DAX (human DAX-1, accession U31929), GAL-ERR2 (human ERR2, Glu 171 - Val 433, accession X51417), GAL-CoA (human SRC-1 Asp 617 - Asp 769, accession U59302).

The RXR LBD expression construct L-RXR contains the SV40 TAg nuclear localization signal (APKKKRKVG (SEQ ID NO: 1)) fused upstream of the human RXRα LBD (Glu 203 - Thr 462). VP-L-FXR contains the 78 amino acid Herpes virus VP16 transactivation domain linked to the amino terminal end of the rat FXR LBD (Leu 181 - Gln 469). CMX-βgal, used as a control gene for comparison with the activation of the receptor or receptor domain being tested, contains the E. coli β-galactosidase coding sequences derived from pCH110 (accession U02445). This gene was conveniently used here, however any unrelated gene which is available and for which a convenient assay exists to measure its activation may be used as a control with the methods of this invention.

RXRm is a human 9-cis retinoic acid receptor ligand binding domain which contains a single point mutation (Asp 322→Pro). This mutated receptor domain retains the ability to bind to DNA and to form heterodimeric complexes with FXR, however it lacks the ability to respond to low concentrations of ligand as the wild-type receptor domain does. This defective LBD allowed the activation assay to screen specifically for compounds which bind and activate the bile acid nuclear receptor (FXR) and not compounds acting through the RXR portion of the heterodimer.

To determine which orphan receptor or receptors were activated by bile and could be exogenously manipulated to modify transcription of bile acid responsive genes, the transfected cells were treated with porcine bile extract. The bile extract was prepared as follows. Bile (Sigma, 1g) was dissolved in water and adjusted to pH 4.0. The water-insoluble material was further extracted with methanol. Methanol-soluble material was dried and redissolved at 100 µg/ml.

CV-1 cells for the activation assays were grown in Dulbecco's modified Eagle's medium supplemented with 10% resin charcoal-stripped fetal bovine serum, 50 U/ml penicillin G and 50 µg/ml streptomycin sulfate (DMEM-FBS) at 37°C in 5% CO₂. One day prior to transfection, cells were plated to 50-80% confluence using phenol red free DMEM-FBS.

The cells were transiently transfected by lipofection. Reporter constructs (300 ng/10⁵ cells) and cytomegalovirus-driven expression vectors (20-50 ng/10⁵ cells) were added, with CMX-β-gal (500 ng/10⁵ cells) as an internal control. After 2 hours, the liposomes were removed and the cells were treated for approximately 45 hours with phenol red free DMEM-FBS containing the test bile acid and other compounds.

Any compound which is a candidate for activation of the bile acid nuclear receptor may be tested by this method. Generally, compounds are tested at several different concentrations to optimize the chances that activation of the receptor will be detected and recognized if present. After exposure to ligand, the cells were harvested and assayed for β-galactosidase activity (control) and activity of the specific reporter gene. All assays disclosed here were performed in triplicate and varied within experiment less than 15%. Each experiment was repeated three or more times with similar results.

Activity of the reporter gene can be conveniently normalized to the internal control and the data plotted as fold activation relative to untreated cells. See Figure 1 for data showing the activation of orphan receptors by bile extract. As shown in the Figure, the bile extract was a strong activator (56-fold) of FXR but had little or no effect on the other orphan receptors tested.

As discussed above, FXR binds to its response element as a heterodimer with RXR (9-cis retinoic acid receptor). This heterodimer can be activated by farnesoid ligands or by RXR-binding ligands (Forman et al., Cell 803-812 (1995); Zavaki et al., Proc. Natl. Acad. Sci. (USA), 94:7909-7914 (1997)). Because the activation of the FXR-RXR heterodimers by bile extract could reflect the presence of ligands for either FXR or RXR, an FXR-RXR complex that is defective in its response to RXR ligands was created to screen for FXR-specific activators. The availability of an RXR ligand-binding domain permits the creation of a screening assay which detects activation of the bile acid nuclear receptor in the absence of RXR effects, preventing false positive results which would otherwise occur.

For the RXR mutant to function in this procedure, the receptor should be minimally activated by RXR ligands and fail to recruit coactivator when exposed to RXR ligands, but retain the ability to dimerize with FXR and to bind DNA as a heterodimer with FXR. Finally, the mutant should not substantially interfere with the normal activity of the bile acid nuclear receptor. To ensure these qualities, tests were performed on the mutant ligand binding domain as described below. Other mutants also can be tested in the same way to determine their suitability for use in the methods of this invention.

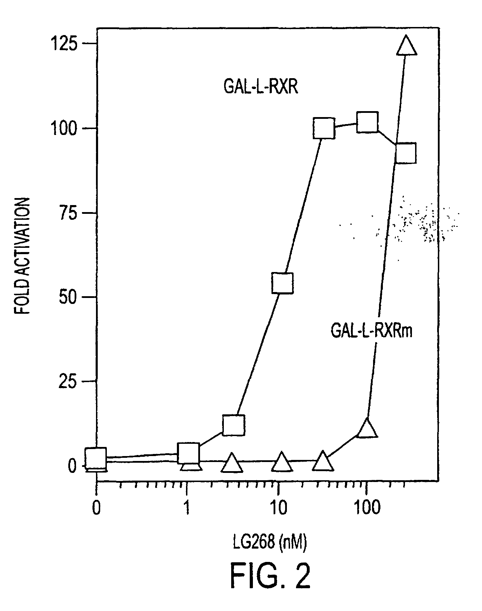

An RXR mutant (RXRm) containing a single point mutation in the LED (Asp 322→Pro) has been found to function particularly well in these analyses. Chimeric receptors containing the yeast GAL4 DNA binding domain fused to the ligand-binding domain of either wild-type RXR (GAL-L-RXR) or RXRm (GAL-L-RXRm) were tested for a response to a synthetic RXR-specific ligand (LG268, 6-[1-(3,5,5,8,8-pentamethyl-5,6,7,8-tetrahydronaphthalen-2-yl)cyclopropyl]nicotinic acid) in the same way as described for the data in Figure 1 for activation of the orphan receptors by bile acids. CV-1 cells were transiently transfected with CMX-βgal, UAS_{G} x 4 and either GAL-L-RXR or the GAL-L-RXRm LBD mutant. After transfection, cells were treated with the concentrations of LG268 indicated in Figure 2. Dimers having the mutant RXR ligand binding domain demonstrated a 10-fold decrease in their potency of activation over dimers having a wild-type RXR ligand binding domain. See Figure 2. Similar results were observed with full-length RXR and RXRm receptors (data not shown).

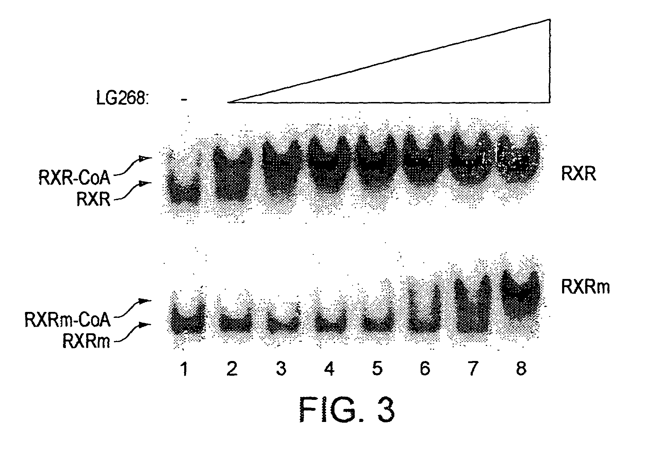

To further confirm the suitability of this mutant, the ability of wild-type RXR and RXRm to bind DNA and recruit coactivator in response to ligand was compared. Electrophoretic mobility shift experiments were performed by first incubating together a mixture of 1.2 µl *in vitro* translated RXR (Figure 3, top panel) or RXRm (Figure 3, bottom panel), 5 µg of purified recombinant GST-GRIP1 coactivator (described below), and a ³²P-labeled DR1 probe (5'-AGCTACCAGGTCAAAGGTCACGTAGCT; SEQ ID NO: 2) with increasing amounts of the RXR ligand LG268 (0-1000 nM). The DR1 probe of SEQ ID NO: 2 was used for all RXR homodimer tests disclosed here. Any nucleic acid probe which is substantially homologous to the DNA-binding domain target sequence may be used for such assays, as long as the ligand-occupied heterodimer binds to the probe with sufficient avidity for the detection method used. Likewise, any convenient label for the nucleotide probe sensitive enough to detect the presence of complexes in the mixture is contemplated for use with the inventive methods.

During incubation, complexes form in which dimers recruit coactivator and bind to the labeled DNA probe. After incubation, the mixture is subjected to electrophoresis under nondenaturing conditions. For this assay, the complexes were electrophoresed through a 5% polyacrylamide gel in 45 mM Tris-base buffer, containing 45 mM boric acid and 1 mM EDTA at room temperature. The gel was subjected to autoradiography to detect the labeled complexes and other components.

In Figure 3, CoA indicates a GST-fusion containing the three receptor interaction domains from the coactivator GRIP1. The electrophoretic mobility shift results indicate that RXRm recruits coactivator with a 100-fold decrease in potency compared to wild type RXR. While both mutant and wild-type receptors bound DNA (Figure 3, lane 1), RXRm failed to recruit coactivator at concentrations that were sufficient for maximal recruitment by the wild-type receptor (Figure 3, compare upper and lower panels, lanes 2-6).

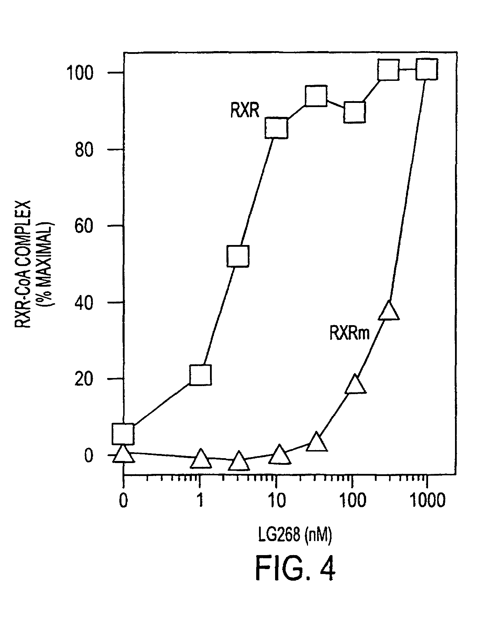

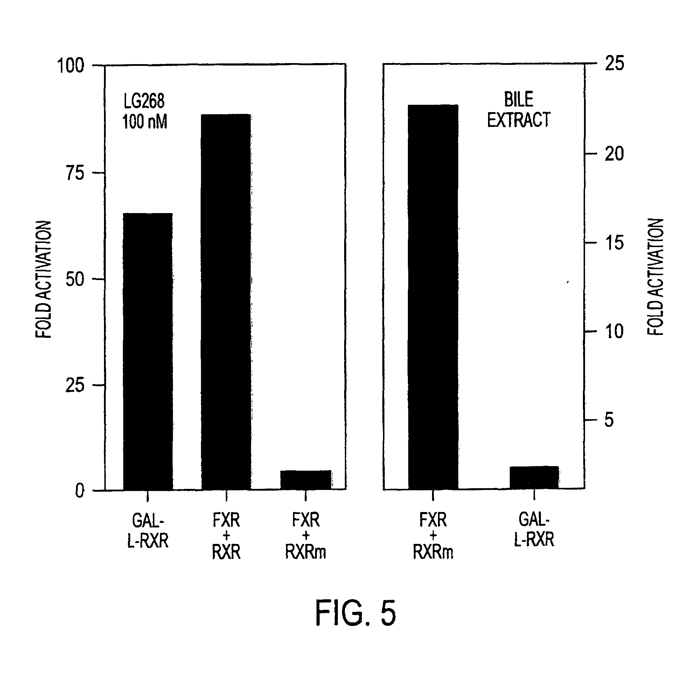

Quantitation of the amount of RXR-coactivator complex shown in Figure 3 was determined by phosphorimager analysis of the autoradiogram and plotted as a function of the LG268 concentration. The data indicated that recruitment of coactivator by RXRm required approximately 100-fold higher concentrations of ligand (Figure 4). Since the RXR mutant retained the ability to bind DNA as a heterodimer with FXR (Figure 5 and data not shown), but lacked the ability to respond strongly to low concentrations of ligand, FXR-RXRm heterodimers could be used to verify activation of the FXR subunits in tests with various ligands. Such heterodimers therefore may advantageously be employed in a method to screen for compounds which activate the FXR nuclear bile acid receptor and thus for compounds able to modify transcription of genes regulated by the receptor.

Confirmation of the analytical procedure was achieved by testing FXR-RXR and FXR-RXRm dimers for activation by RXR and FXR ligands. CV-1 cells were transfected with plasmids harboring the receptor domains indicated in Figure 5 and treated with either 100 nM LG268 (left panel) or a methanol extract of .porcine bile (200 µg/ml, right panel). While LG268 activated RXR (GAL-L-RXR) and FXR-RXR heterodimers, FXR-RXRm showed little or no response to the RXR-specific ligand LG268 (Figure 5, left panel). In contrast, the bile extract retained the ability to activate FXR-RXRm but had little effect on GAL-L-RXRm (Figure 5, right panel). Similar results were obtained when RXRm was replaced with a different RXR mutant containing a defective AF2 transactivation domain (Phe 450→Ala, Schulman et al., Mol. Cell. Biol., 16:3807-3813 (1996)) (data not shown). This type of assay therefore can specifically discriminate between activation by RXR ligands and FXR ligands. These particular data indicate that bile extract contains an FXR-specific activator.

Further data showed.that activation requires the AF2 transactivation domain of FXR (data not shown). In addition, bile acids induced activation with the expected kinetics (activity is observed within one hour of ligand addition to cells; data not shown). Taken together, the data provided herein demonstrate that FXR is the endogenous bile acid sensor which can be manipulated exogenously with appropriate ligands to modify the regulation of genes dependent on activation via FXR, such as important genes involved in the control of cholesterol metabolism.

A chemical fractionation scheme was devised to identify and purify the biliary component in the bile extract which binds to and activates FXR. As an initial step, the methanol-water bile extract was fractionated by silica gel chromatography. Briefly, the extract was applied to a column and successively eluted with chloroform-methanol at ratios of 8:1 and 4:1, then with 100% methanol. Fifty-six fractions were collected, pooled and tested for their ability to activate FXR-RXRm. The active fraction was further purified by preparative thin layer chromatography (PTLC) and separated into 5 fractions (A-E).

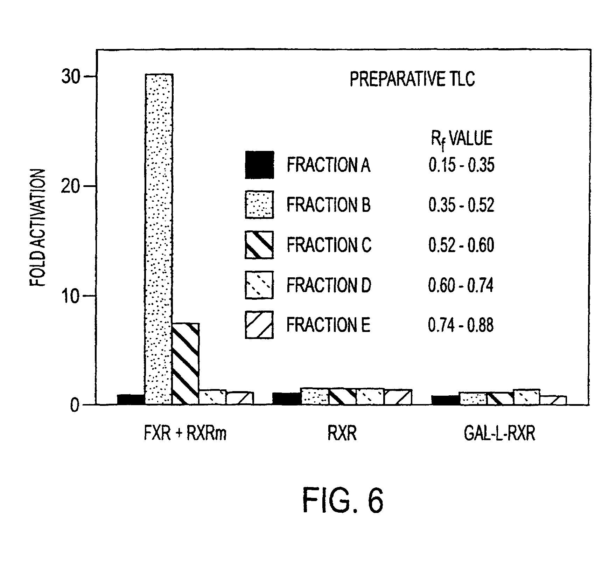

To test for FXR activation by material in these fractions, CV-1 cells were transfected with plasmids harboring the receptor domains indicated in Figure 6 and treated with 25 µg/ml of each of the 5 PTLC fractions. Fraction B (PTLC 0.35<R_{f}<0.52) was the most active (30-fold greater activation of FXR-RXRm relative to activation of RXR). See Figure 6.

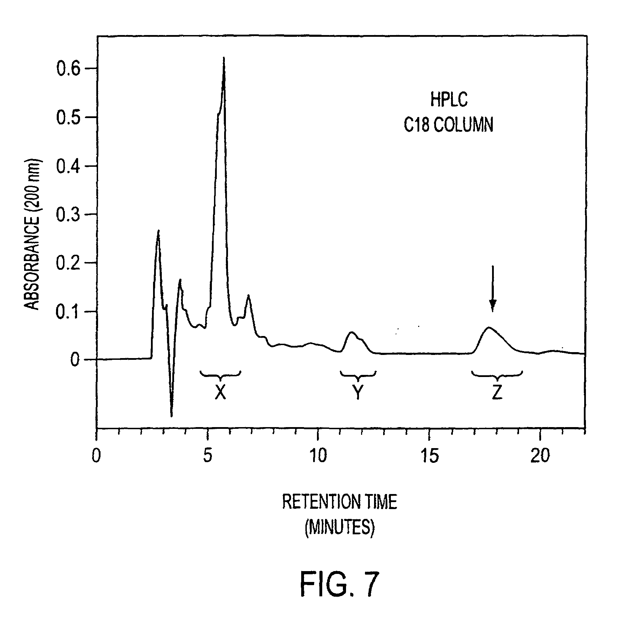

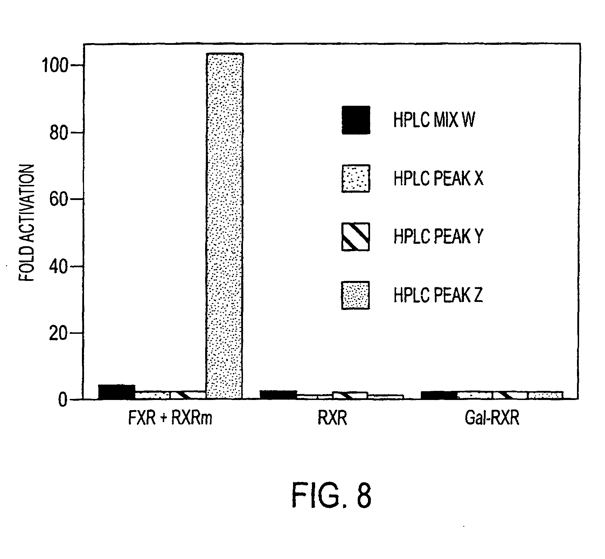

The active material of PTLC fraction B was further purified by reverse-phase HPLC on a C18 column. Absorbance was monitored at 200 nm. Three main peaks were resolved (peaks X, Y and Z; Figure 7). These three peaks were collected in isolation. The remaining fractions were pooled to form a fourth fraction (W). The four fractions were tested for activation of FXR-RXRm as above. Briefly, CV-1 cells were transfected with plasmids harboring the receptor domains indicated in Figure 8 and treated with each of the HPLC fractions at concentrations of 25 µg/ml. Fractions W, X and Y had little or no activity (Figure 8). Remarkably, peak Z induced a dramatic 102-fold activation of FXR-RXRm but had no effect on RXR. These data indicate that the material in peak Z not only potently activated FXR, but did not contain any RXR activating material. Thus, the material in peak Z was selected for structural analysis.

After methylation of the material in peak Z, tandem gas chromatography-mass spectrometry (GC-MS) was performed. The gas chromatogram indicated that peak Z contained one predominant peak, indicating that the active component had been purified to near homogeneity. Compound Z had a retention time of 14.41 minutes in this assay and was indistinguishable from a synthetic chenodeoxycholic acid (CDCA) standard. The mass-spectrum of the material of peak Z also was indistinguishable from that of a chenodeoxycholic acid (CDCA) standard. To further confirm the identity of this material, ¹³C-NMR, ¹H-NMR, DEPT, DQFCOSY and HMQC spectra (data not shown) were obtained and found to be identical to the CDCA standard. The component in porcine bile extract which activates the bile acid nuclear receptor in this assay therefore was identified as CDCA.

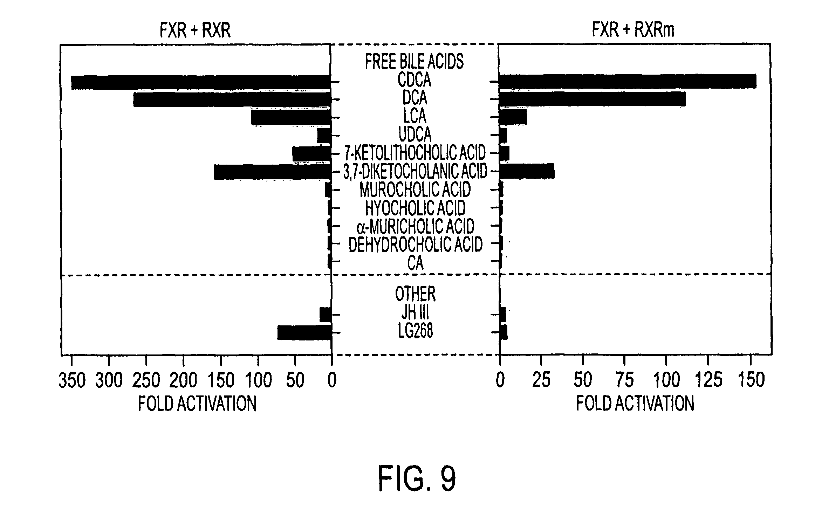

A variety of commercially available bile acids (Sigma) were tested for their ability to activate known FXR-RXR (Figure 9; left panel) and FXR-RXRm (Figure 9, right panel). The RXR ligands LG268 and juvenile hormone III were also included as test ligands for comparison. For these assays, CV-1 cells were transfected with CMX-βgal, EcRE x 6 and FXR + RXR (left panel) or FXR + RXRm (right panel) and treated with the indicated bile acids (100 µM), juvenile hormone III (JH III, 50 µM) or LG268 (100 nM). Bile acids are denoted in the Figure as follows: CA, cholic acid; CDCA, chenodeoxycholic acid; DCA, deoxycholic acid; LCA, lithocholic acid; UDCA, ursodeoxycholic acid. As expected from the studies of bile acid extract, synthetic CDCA proved to be a highly effective activator of FXR (346-fold activation; Figure 9, left panel). CDCA failed to activate other receptors, including RXRα; PPARα, γ and δ; VDR; T₃Rβ; RAR; PXR; LXRα and CARβ (data not shown).

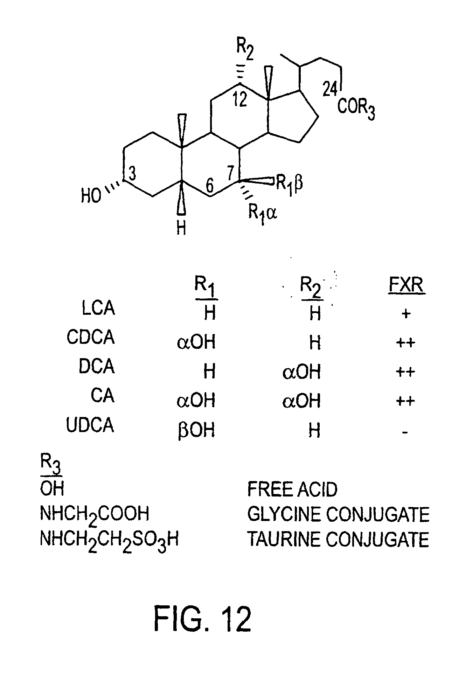

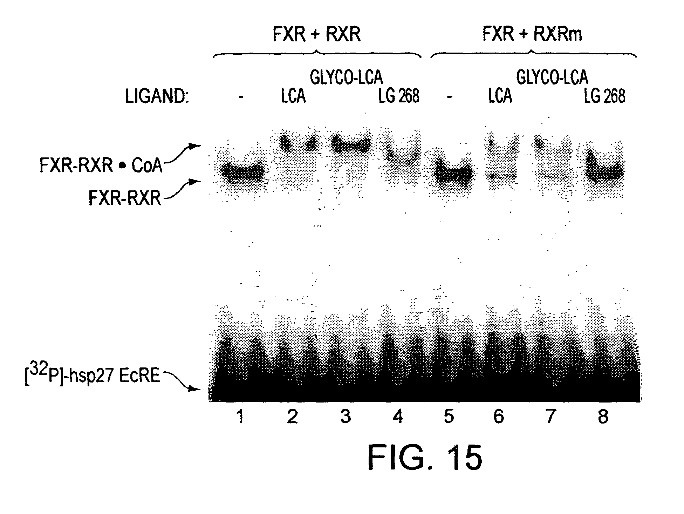

The secondary bile acids, CDA and LCA, were also highly effective, inducing 246- and 106-fold activations of FXR-RXR, respectively. Qualitatively similar results were seen with FXR-RXRm (Figure 9, right panel), indicating that all of these bile acids act through the FXR subunit. Ursodeoxycholic acid (UDCA, 5β-cholanic acid-3α,7β-diol), the 7β-epimer of CDCA, was inactive while substitution of a hydroxyl group with a ketone at the 7-position produced a compound (7-ketolithocholic acid, 5β-cholanic acid-3α-ol-7-one) with activity intermediate between CDCA and UDCA Figures 9, 15). Thus, the configuration around the 7 position is a crucial determinant of FXR activity with 7α-OH » 7-keto » 7β-OH, Comparison of 7-ketolithocholic acid and 3,7-diketocholanic acid (5β-cholanic acid-3,7-dione) suggests that a ketone in the 3-position is preferred to a 3α-hydroxyl group. Several di- and tri-hydroxy bile acids with a hydroxyl group in the 6 position were inactive (murocholic acid, hyocholic and α-muricholic acid) as was dehydrocholic acid (5β-cholanic acid-3,7,12-trione). Taken together, these data suggest that 3,7- and 3,12-substituted bile acids are highly effective activators of FXR. Figure 12 is a summary comparison of the chemical structure of key bile acids and their efficacy as FXR activators.

Since farnesoid metabolites had previously been shown to activate FXR (Forman et al., Cell, 81:687-693 (1995)), the activity of one of the most active farnesoid activators, juvenile hormone III (JH III, 50 µM) was tested. This compound was active, but had a far weaker activity relative to the most efficacious bile acids (Figure 9).

CDCA and CA are both major bile acids produced via the classical pathway, however although CDCA was an extremely effective activator of FXR, CA was inactive. Both CA and conjugated bile acids are relatively hydrophilic compounds that do not readily cross cell membranes. It was possible that no activation of the bile acid nuclear receptor was detected in the assay not because the compounds themselves were not active, but simply because they could not enter the cells in a high enough concentration. A second assay for bile acid nuclear receptor activation was devised which could effectively test for activation by compounds which cannot cross the cell membrane unassisted.

The liver and ileum express tissue-specific bile acid transport proteins for efficient uptake of these compounds. (Craddock et al., Am. J. Physiol., 274:G157-169 (1998)). Neither of these transporters were expressed in the CV-1/COS cells used above. CV-1 cells therefore were co-transfected with a human liver bile acid transporter (accession L21893). Use of this transporter allows selection of bile acid or bile acid-derived compounds or compounds which due to their structural similarity to bile acid are transported by the transporter, which due to their hydrophilic nature do not easily enter cells, to be tested for activation of the intracellular bile acid nuclear receptor. Bile acid transporters from renal or ileal tissues may also be used efficaciously. Any suitable non-specific transporter may also be used.

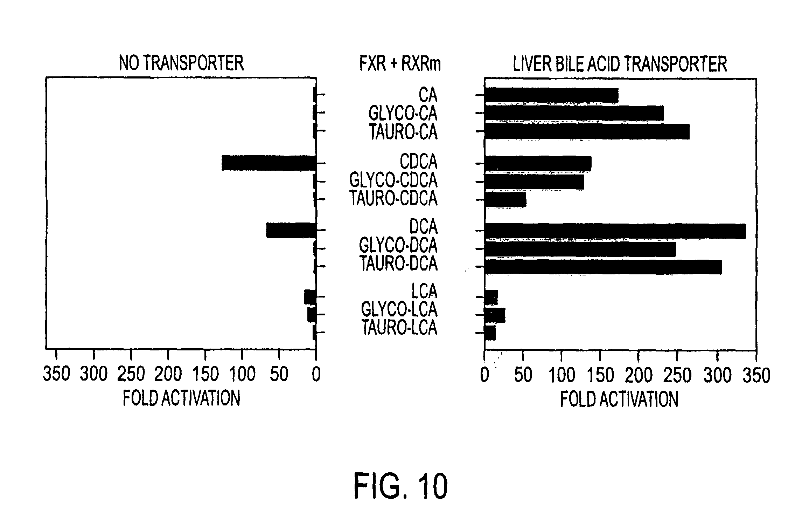

For assay of bile acid nuclear receptor activation by hydrophilic bile acids, CV-1 cells were transfected with CMX-βgal, EcRE x 6, and FXR + RXR alone (Figure 10, left panel) and additionally with the liver bile acid transporter (Figure 10, right panel). After transfection, cells were treated with 100 µM concentrations of the indicated bile acid.

Although CA was inactive in the absence of a bile acid transporter (Figure 10, left panel), coexpression of the liver bile acid transporter allowed CA to exhibit a dramatic 170-fold activation of FXR (Figure 10, right panel). Similarly, while the glycine and taurine conjugates of CA, CDCA, DCA and LCA were weak or inactive in the first assay, these more hydrophilic bile acids were highly effective in cells expressing the liver bile acid transporter (Figure 10, compare left and right panels). Similar results were seen with the ileal-specific bile acid transporter (data not shown). Thus, this assay was able to demonstrate that intracellular CA is an effective FXR activator as are the glycine and taurine conjugates of active free bile acids. The assay can be used to test both compounds transported by the bile acid transporter and compounds which are not. The results also demonstrate that FXR and the bile acid transporters share an overlapping specificity.

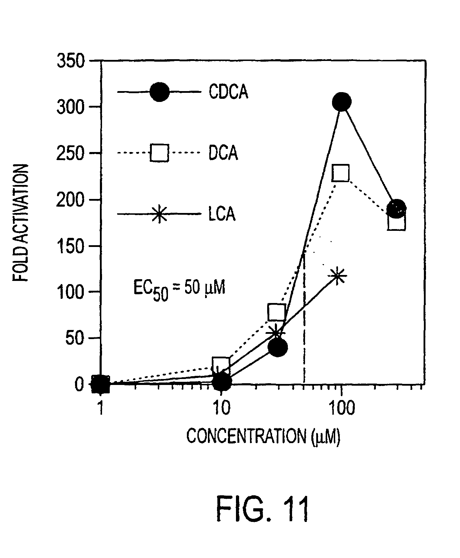

In addition to the structure-activity studies, dose-response analyses were performed for some bile acid nuclear receptor ligands. For these analyses, shown in Figure 11, CV-1 cells were transfected as above, with the liver bile acid transporter, and treated with the indicated varying concentrations of each bile acid. CDCA, DCA and LCA each displayed an EC₅₀ of approximately 50 µM. The structure-activity relationship (Figures 9, 10 and 12) and dose-response profile (Figure 11) of bile acids for FXR are similar to that reported for the endogenous bile acid sensor (Chiang, Front. Biosci, 3:D176-193 (1998); Kanda et al., Biochem. J., 330:261-265 (1998); Twisk et al., Biochem. J., 305:505-511; Zhang et al., J. Biol. Chem., 273:2424-2428 (1998)). This validates the model used in these studies, showing that the results determined by this assay correlate well with in vivo results.

In addition, the EC₅₀ of bile acids for the bile acid nuclear receptor and the physiologic concentration of the bile acids are closely correlated. For example, the transcriptional effects of CDCA and DCA occur at concentrations of about 50-250 µM (Kanda et al., Biochem. J., 330:261-265 (1998); Twisk et al., Biochem. J., 305:505-511 (1995); Zhang et al., J. Biol. Chem., 273:2424-2428 (1998)). This concentration is very close to the EC₅₀ discovered here for the bile acid nuclear receptor (50 µM) and matches the endogenous concentration of these compounds in bile (CDCA: 10-150 µM; DCA 5 µM) (Matoba et al., J. Lipid Res., 27:1154-1162 (1986)) and intestinal fluid (CDCA: 50 µM; DCA: 320 µM; LCA: 120 µM) (McJunkin et al., Gastroenterol., 80:1454-1464 (1981)). The EC₅₀ for the bile acid nuclear receptor also matches the reported Michaelis constant (Kₘ) of 3-100 µM for liver and ileal bile acid transport proteins (Boyer et al., Am. J. Physiol., 266:G382-G387 (1994); Wong et al., J. Biol. Chem., 269:1340-1347 (1994)). Indeed, the bile acid nuclear receptor responds effectively to bile acids at intracellular concentrations established by the bile acid transporters. See Figure 10, right panel.

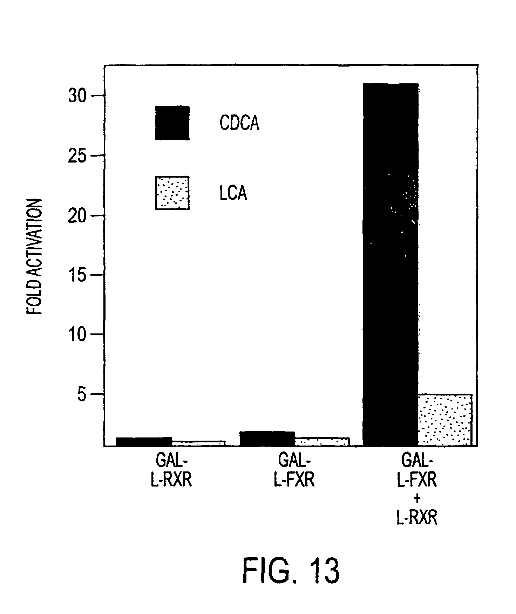

As discussed above, classical nuclear receptors contain modular LBDs that confer ligand-responsiveness to heterologous DNA binding domains. To test whether the bile acid nuclear receptor also requires an interaction with a heterodimeric partner for high affinity binding of the endogenous ligand, the ability of a CDCA and LCA to activate the receptor was tested in cells co-expressing GAL-L-RXR, GAL-L-FXR or GAL-L-FXR plus L-RXR. As expected, CDCA and LCA did not activate the GAL-L-RXR chimera. See Figure 13. Coexpression of GAL-L-FXR along with the RXR LBD (L-RXR), however, resulted in a complex that was responsive to both CDCA and LCA (Figure 13). Cells expressing only one LBD (either RXR or FXR) were not activated by either bile acid. These data make it clear that not only is bile acid responsiveness mediated by the FXR LBD, requiring an intact FXR AF2 transactivation domain, but activation of the receptor requires an association with its dimerization partner. In addition, time course experiments indicated that LCA and CDCA activate FXR with the kinetics expected for nuclear receptor ligands, i.e., activity is observed within 1 hour of addition to cells (data not shown).

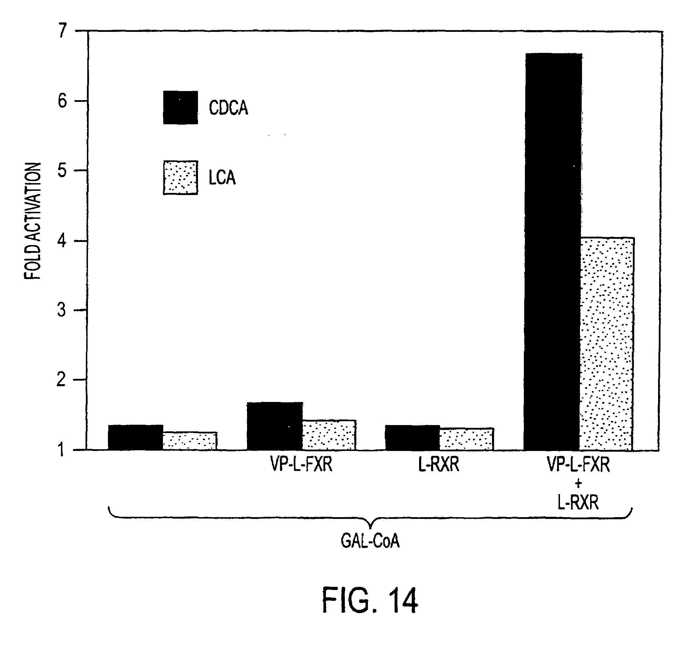

To assess the ability of CDCA and LCA to induce coactivator recruitment, CV-1 cells were transfected with CMX-βgal, UAS_{G} x 4 and GAL-CoA (a GAL4 fusion construct containing the 3 receptor interaction domains of the coactivator SRC-1). Where indicated in Figure 14, cells also were cotransfected with constructs containing the ligand binding domain of RXR (L-RXR) and/or the VP16 transactivation domain fused to the ligand binding domain of FXR (VP-L-FXR). After transfection, cells were treated with 100 µM CDCA or LCA. Neither CDCA nor LCA were able to promote a functional interaction between a GAL4-coactivator fusion protein (GAL-CoA) and a chimera containing the VP16 transactivation domain fused to the FXR LBD (VP-L-FXR). However, in the presence of RXR LBD, the bile acids induced a 4-7 fold increase in activity (Figure 14).

Coactivator recruitment assays have become established as a reliable method to identify and test the activity of orphan receptor ligands (Blumberg et al., Genes Dev., 12:1269-1277 (1998); Forman et al., Nature, 395:612-615 (1998); Kliewer et al., Cell, 92:73-82 (1998); Krey et al., Mol. Endocrinol., 11:779-791 (1997). In accordance with the present invention, a mammalian two-hybrid in vitro coactivator recruitment assay was developed to examine whether putative ligands could promote a functional association between FXR and a coactivator as a test of a ligand's ability to modify the transcription of genes regulated by the bile acid nuclear receptor.

In vitro coactivator recruitment assays were performed by adding the ligand to a mixture of the following components: bile acid nuclear receptor, 9-cis retinoic acid receptor, a coactivator, labeled bile acid nuclear receptor response element (probe). A polyamino acid containing the receptor interaction domains of co-activator GRIP1 may be used as the coactivator. Any functional coactivator or coactivator complex is contemplated for use in this assay. GRIP1 was expressed in bacteria and purified for these assays. The GST-GRIP1 construct, containing the three receptor interaction domains of mouse GRIP1 (Arg 625 - Lys 765, accession U39060) fused to glutathione-S-transferase, was created for bacterial expression of the GRIP1 coactivator. Other suitable coactivators are known in the art, for example PBD/DRIP 205/TRAP 220, and may be used with the inventive methods disclosed here. Response elements suitable for use in this assay may be any nucleic acid probe which is substantially homologous to the target DNA sequence of the bile acid nuclear receptor.

Any response element compatible with the assay system may be used. Oligonucleotide sequences which are substantially homologous to the DNA binding region to which the nuclear receptor binds are contemplated for use with the inventive methods. Substantially homologous sequences (probes) are sequences which bind the ligand activated receptor under the conditions of the assay. Response elements can be modified by methods known in the art to increase or decrease the binding of the response element to the nuclear receptor.

The following response elements were used in the specific assays exemplified here: hsp27 EcRE x 6 (Yao et al., Nature, 366:476-479 (1993)), UAS_{G} x 4, PPRE x 3 (Forman et al., Cell, 81:687-693(1995)), βRE2 x 3 (Forman et al., Nature, 395:612-615 (1998)), LXRE x 3 (Willy et al., Genes Dev., 9:1033-1045 (1995)), T₃RE (MLV) x 3 (Perlmann et al., Genes Dev., 7:1411-1422 (1993)), SF1 x 4 (5'-AGCTTAGCCAAGGTCAGAGAAGCTT; SEQ ID No: 3) and DR0 x 2 (5'-AAGCTTCAGGTCAAGGTCAGAGAGCTT; SEQ 10 No: 4).

After addition of the putative ligand to the mixture of components describe above and mixing, the mixture is incubated under conditions. The formation of complexes in the mixture were analyzed by electrophoretic mobility shift, as shown in Figure 15, however, any method of separating the complexes formed in the mixture from the individual components may be used, so long as it is sufficient to resolve the labeled complexes from the other components in the mixture. Techniques such as, for example, thin layer chromatography, high pressure liquid chromatography, size exclusion chromatography, sedimentation, immunoseparation techniques, or any other convenient method known in the art may be used.

As expected, FXR-RXR heterodimers failed to recruit coactivator in the absence of ligand (Figure 15, lane 1). Important in the data provided in Figure 15, the addition of LCA shifted the majority of the heterodimer into a complex with the coactivator GRIP1 (lane 2). Similar results were seen with glyco-LCA (lane 3) and with LG268 (lane 4). To distinguish between binding through the FXR and RXR subunits, the coactivator recruitment assays were repeated substituting RXRm for RXR. See Figure 15, lanes 5-8. Significantly, both LCA (lane 6) and glyco-LCA (lane 7) recruited coactivator, while LG268 was inactive (lane 8). These in vitro results demonstrate that LCA and its glycine conjugate are FXR-specific ligands.

While LCA and glyco-LCA were active in the in vitro coactivator recruitment assay, a standard probe of ligand binding activity, other active bile acids including CA, CDCA and DCA were less effective in recruiting GRIP1 or the related coactivators SRC-1 and ACTR (data not shown). Based on their shared structures, activities and activation kinetics, CA, CDCA and DCA are all FXR ligands though they may also utilize one of the many other nuclear receptor coactivators that have been recently described. See, for example, Blanco et al., Genes Dev. 12:1638-1651 (1998); Fondell et al., PNAS USA 96:1959-1964 (1999).

The coactivator recruitment assay efficiently detected compounds which were able to form a functional binding relationship with the response element of DNA which regulates a bile acid nuclear receptor target gene. Bile acids can inhibit transcription of several genes, including Cyp7a and sterol 27-hydroxylase. (Chiang, Front. Biosci 3:D176-193 (1998)). In addition to being inhibited by its bile acid end-products, Cyp7a transcription is stimulated by the accumulation of its substrate, cholesterol. This response to cholesterol is mediated by the oxysterol receptor, LXRα (Peet et al., Cell 93:693-704 (1998)). Thus, control of cholesterol catabolism to bile acids through the Cyp7a pathway is subject to positive feedback by cholesterol and negative feedback by bile acids, as illustrated by the following diagram: 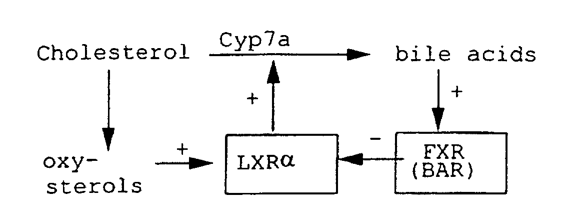 As is the case for the bile acid nuclear receptor, LXRα utilizes RXR as an obligate dimerization partner. LXRα-RXR heterodimers are constitutively active (Apfel et al., Mol. Cell. Biol. 14:7025-7035 (1994); Forman et al., Proc. Natl. Acad. Sci. USA 94:10588-10593 (1997); Willy et al., Genes Dev. 9:1033-1045 (1995)), presumably due to the presence of endogenous LXR ligands, however they are inactive alone.

FXR and LXRα are co-expressed in several tissues (liver, intestine and kidney). A series of assays therefore were performed to determine whether bile acids have an effect on transcriptional control by LXRα. Similar experiments were performed with the thyroid hormone receptor (T₃R) which also upregulates Cyp7a transcription in the liver (see Crestani et al., Biochem. Biophys. Res. Commun. 198:546-553 (1994)). In receptor activation assays using LXRE X3- TK-Luc, LXRα and RXR exhibited little activity alone, but when expressed together they constitutively activated the LXR reporter (data not shown). Coexpression of RXR had only a slight effect on T₃ responsiveness, which indicates that endogenous levels of RXR are sufficient to support the function of T₃R, unlike LXRα and FXR.

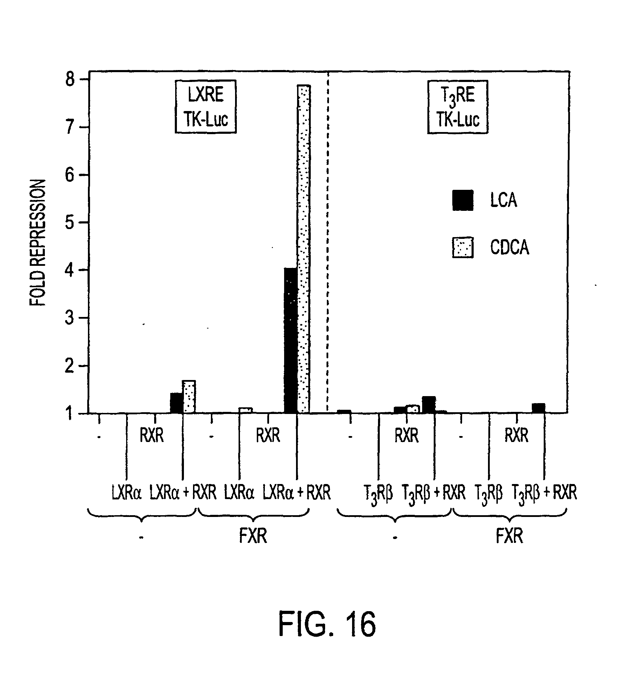

To determine whether ligand occupied bile acid receptor inhibits LXRα, CV-1 cells were transfected with CMX-βgal, LXRE x 3 and expression vectors containing the receptors indicated in Figure 16, left panel. Assays were conducted in the presence or absence of an FXR expression vector. Cells were treated with 0 µM or 100 µM concentrations of LCA or CDCA. Fold repression in the Figure represents inhibition of the constitutive activity of LXR by these bile acid nuclear receptor ligands. The right panel of Figure 16 shows cells transfected as above, substituting T₃R x 3 for LXRE x 3. For these assays, cells were treated with 100 nM T₃, alone or in the presence of 100 µM LCA or CDCA. Fold repression in the right panel represents inhibition of T₃-stimulated activity.

In the absence of the bile acid nuclear receptor, bile acids had little effect on LXRα activity. In its presence however, LCA and CDCA caused a dramatic 4-8 fold repression of LXRα activity. See Figure 16. This inhibition was relieved by addition of LXR ligands (data not shown). Bile acid dependent suppression had no effect on T₃ responsiveness and was specific to LXRα. Thus LXRα and FXR possess opposing metabolic functions with FXR acting as a bile acid sensor that inhibits LXRα in response to physiologically relevant bile acids. Because LXRαa is a positive regulator of Cyp7a transcription, this molecular link between LXRα and FXR signaling provides a mechanism for bile acids to negatively regulate LXRα-dependent genes. The assays described here thus are useful in selecting compounds which can modify the transcription of these genes as well.

This invention provides a method of screening for putative FXR ligands, which may act as agonists or antagonists in the FXR receptor, and therefore may be used therapeutically or prophylactically for the control of cholesterol metabolism.

The assays described above and exemplified below provide methods of selecting compounds which modulate the transcription of genes regulated by the bile acid nuclear receptor. The invention if further described and illustrated in the following examples, which are not intended to be limiting.

### EXAMPLES

### Example 1. Transient Transfection Assay for FXR Activity

CV-1 cells were grown in Dulbecco's Modified Eagle's medium supplemented with 10% resin-charcoal stripped fetal bovine serum, 50 U/ml penicillin G and 50 µg/ml streptomycin sulfate (DMEM-FBS) at 37°C in 5% CO₂. One day prior to transfection, cells were plated to 50-80% confluence using phenol-red free DMEM-FBS. Cells were transiently transfected by lipofection as described (Forman et al., Cell, 81:687-693 (1995). Luciferase reporter constructs (300 ng/10⁵ cells) containing the herpes virus thymidine kinase promoter (-105/+51) linked to the ecdysone response element (EcRE x 6) and cytomegalovirus driven expression vectors (20-50 ng/10⁵ cells) were added, along with CMX-β-gal as an internal control. Mammalian expression vectors were derived from pCMX which contains the cytomegalovirus promoter/enhancer followed by a bacteriophage T7 promoter for transcription in vitro. Two assays were performed in parallel, one using cells transfected with expression vectors containing CMX-β-gal, EcRE x 6, FXR and RXR and one using cells transfected with expression vectors containing CMX-β-gal, EcRE x 6, FXR and RXRm. After incubation with liposomes for 2 hours, the liposomes were removed and cells treated for approximately 45 hours with phenol-red free DMEM-FBS containing 100 µM CDCA. After exposure to ligand, the cells were harvested and assayed for luciferase and β-galactosidase activity according to known methods. All points were assayed in triplicate and varied by less than 15%. Each experiment was repeated three or more times with similar results.

### Example 2. Transient Transfection Assay for FXR Activity Suitable for Hydrophilic Compounds

An assay was performed in Example 1 with the exception that the cells were also co-transfected with a pcDNA expression vector for the human liver bile acid transporter.

### Example 3. Screening Assay for Compounds which Modulate the Transcription of a Bile Acid Nuclear Receptor Target Gene

CV-1 cells are grown in Dulbecco's Modified Eagle's medium (DMEM) supplemented with 10% resin-charcoal stripped fetal bovine serum. 50 U/ml penicillin G and 50 µg/ml streptomycin sulfate at 37°C in 5% CO₂. Cells are plated to 50-80% confluence one day prior to transfection using phenol red-free DMEM-FBS. The cells are transfected by lipofection using N-[1-(2,3-dioleoyloxy)propyl]-N,N,N-ammonium methyl sulfate according to the instructions of the manufacturer (Boehringer Mannheim).

The CV-1 cells are transfected with CMX-βgal, constructs containing the ligand-binding domain of RXR (L-RXR) and the ligand-binding domain of FXR (L-FXR), and a luciferase reporter construct containing the herpesvirus thymidine kinase promoter (-105/+51) linked to the indicated number of copies of the response element hsp27 EcRE x 6. A parallel assay is performed in which the ligand-binding domain of RXR accession no. X52773) is replaced with the ligand-binding domain RXRm. After transfection, cells are treated with varying concentrations of candidate bile acid receptor agonist or antagonist compounds for approximately 45 hours in phenol red free DMEM-FBS. After exposure to the compounds, cells are harvested and assayed for luciferase and β-galactosidase activity.

### Example 4. Screening Assay for Compounds which Modulate the Transcription of a Bile Acid Nuclear Receptor Target Gene

A screening assay is performed according to Example 3 with the exception that the cells are also co-transfected with a pcDNA expression vector for the human liver bile acid transporter.

### Example 5. Screening Assay for Compounds which Modulate the Transcription of a Bile Acid Nuclear Receptor Target Gene

A screening assay is performed according to Example 3 with the exception that the parallel assay using the expression construct containing RXRm is omitted.

### Example 6. Coactivator Recruitment Assay

GST-GRIP1 was expressed in E. coli and purified on glutathione-sepharose columns. In vitro translated FXR + RXR (Figure 15, left panel) or FXR + RXRm (Figure 15, right panel) and GST-GRIP1 (5 µg) were incubated for 30 minutes at room temperature with 100,000 cpm of a Klenow-labeled hsp27 EcRE probe (5'-AGCTCGATGGACAAGTGCATTGAACCCTTGAAGCTT; SEQ ID NO: 5) in 10 mM Tris pH 8, 50 mM KCL, 6% glycerol, 0.05% NP-40, 1 mM DTT, 12.5 ng/µl poly dI·dC and the ligands to be tested for coactivator recruitment. Complexes were electrophoresed through a 5% polyacrylamide gel in 0.5x TBE (45 mM Tris-base, 45 mM Boric Acid, 1 mM EDTA) at room temperature. Electrophoretic mobility indicated recruitment of coactivator.

## Claims

1. A method of screening for compounds useful in modulating FXR-mediated gene transcription which comprises contacting a mixture of FXR and RXR with a compound and determining whether said compound promotes formation of an FXR-RXR heterodimer, wherein the RXR is an RXR mutant (RXRm) which contains a functional DNA-binding domain and which has a mutation in the ligand-binding domain which prevents substantial activation by RXR ligands but which does not otherwise substantially affect the ability of the RXR mutant receptor to form heterodimers with FXR.

2. A method of screening compounds for FXR antagonist activity which comprises contacting a mixture of FXR and RXR with a compound and determining whether said compound inhibits the agonist-promoted formation of an FXR-RXR heterodimer, wherein the RXR is an RXR mutant (RXRm) which contains a functional DNA-binding domain and which has a mutation in the ligand-binding domain which prevents substantial activation by RXR ligands but which does not otherwise substantially affect the ability of the RXR mutant receptor to form heterodimers with FXR.

3. The method of claim 2, in which the mixture further contains a known FXR agonist.

4. A method for screening compounds for cholesterol catabolism-modulating activity which comprises:
(1) providing a first mixture which contains
(i) an FXR receptor,
(ii) an RXR receptor,
(iii) a labelled DNA probe which contains a sequence of nucleotides to which the DNA-binding domain of a ligand-FXR-RXR complex specifically binds;
(2) providing a second mixture which contains
(i) an FXR receptor,
(ii) an RXR mutant receptor ("RXRm") which contains a functional DNA-binding domain and which has a mutation in the ligand-binding domain which prevents substantial activation by RXR ligands but which does not otherwise substantially affect the ability of the RXR mutant receptor to form heterodimers with FXR or of such heterodimers to recruit coactivator,
(iii) a labelled DNA probe which contains a sequence of nucleotides to which the DNA-binding domain of a ligand-FXR-RXR complex specifically binds;
(3) contacting said first and second mixtures with the compound being screened;
(4) determining whether the compound causes binding of an FXR-RXR heterodimer to the DNA probe; and
(5) determining whether the compound being screened causes binding of an FXR-RXRm heterodimer to the DNA probe.

5. The method of claim 4, which further comprises contacting the first and second mixtures with a known FXR ligand and selecting compounds that inhibit the ability of said known FXR ligand to cause the FXR-RXR heterodimer to bind to the DNA probe.

6. The method of claim 5, which further comprises selecting compounds that do not cause substantial binding of the FXR-RXRm heterodimer to the DNA probe.

7. A method of screening for compounds useful in modulating FXR-mediated gene transcription which comprises contacting a mixture of FXR, RXR and an FXR-RXR coactivator with a compound and determining whether said compound promotes coactivator recruitment by an FXR-RXR heterodimer, wherein the RXR is an RXR mutant (RXRm) which contains a functional DNA-binding domain and which has a mutation in the ligand-binding domain which prevents substantial activation by RXR ligands but which does not otherwise substantially affect the ability of the RXR mutant receptor to form heterodimers with FXR or of such heterodimers to recruit coactivator.

8. A method of screening compounds for FXR antagonist activity which comprises contacting a mixture of FXR, RXR, an FXR-RXR coactivator and a known FXR agonist with a compound and determining whether said compound inhibits the agonist-promoted coactivator recruitment by an FXR-RXR heterodimer, wherein the RXR is an RXR mutant ("RXRm") which contains a functional DNA-binding domain and which has a mutation in the ligand-binding domain which prevents substantial activation by RXR ligands but which does not otherwise substantially affect the ability of the RXR mutant receptor to form heterodimers with FXR or of such heterodimers to recruit coactivator.

9. The method of any of claims 1 to 8 in which the RXRm is an Asp332→Pro mutant of human RXRα.

10. The method of claim 7 or 8 in which the coactivator is a polypeptide or active fragment thereof which contains a peptide motif that interacts with the FXR-RXR heterodimer in a ligand-dependent manner.

11. The method of claim 7 or 8 in which the coactivator is selected from CBP, SRC-1 and PDB/DRIP205/TRAP220.

12. The method of claim 7 or 8 in which the coactivator is GRIP1.

13. The method of claim 4, 5 or 6 in which the DNA probe comprises the nucleotide sequence of SEQ ID NO. 3 or SEQ ID NO. 4.

14. A method of screening for compounds useful in modulating FXR-mediated gene transcription, which comprises:
(a) transfecting mammalian cells with a gene encoding FXR under control of an operative promoter;
(b) transfecting said cells with an operative reporter gene under control of a promoter linked to a DNA sequence which encodes an operative response element to which ligand-activated FXR or FXR complex binds to initiate transcription of said reporter gene;
(c) culturing said cells in the presence of a compound being screened; and
(d) monitoring said cells for transcription or expression of the reporter gene as an indication of FXR activation, wherein said cells are transfected with a gene encoding a bile acid transporter molecule under control of an operative promoter.

15. The method of claim 14, wherein said cells are cultured in the presence of a known FXR ligand and the diminution of transcription or expression of the reporter gene is an indication that the compound being screened is an FXR antagonist.

16. The method of claim 15 in which the method is used to identify compounds that are useful for increasing cholesterol catabolism.

## Patentansprüche

1. Verfahren zum Screenen von zur Modulation der FXR-vermittelten Gentranskription nützlicher Verbindungen, das das in Kontakt bringen einer Mischung von FXR und RXR mit einer Verbindung und die Bestimmung umfasst, ob diese Verbindung die Bildung eines FXR-RXR Heterodimers fördert, worin RXR eine RXR-Mutante (RXRm) ist, die eine funktionelle DNA-Bindungsdomäne enthält und die eine wesentliche Aktivierung durch RXR-Liganden verhindert, die aber ansonsten die Fähigkeit des mutierten RXR-Rezeptors zur Bildung von Heterodimeren mit FXR nicht wesentlich beeinflusst.

2. Verfahren zum Screenen von Verbindungen mit FXR-Antagonist-Aktivität, das das in Kontakt bringen einer Mischung von FXR und RXR mit einer Verbindung und die Bestimmung umfasst, ob diese Verbindung die agonist-geförderte Bildung eines FXR-RXR-Heterodimers hemmt, worin RXR eine RXR-Mutante (RXRm) ist, die eine funktionelle DNA-Bindungsdomäne enthält und die eine Mutation in der Ligandenbindungsdomäne hat, die eine wesentliche Aktivierung durch RXR-Liganden verhindert, die aber ansonsten die Fähigkeit des mutierten RXR-Liganden zur Bildung von Heterodimeren mit FXR nicht wesentlich beeinflusst.

3. Verfahren nach Anspruch 2, worin die Mischung weiterhin einen bekannten FXR-Agonisten enthält.

4. Verfahren zum Screenen von Verbindungen auf Cholesterinabbau modulierende Aktivität umfassend
(1) Bereitstellung einer ersten Mischung, die
(i) einen FXR-Rezeptor,
(ii) einen RXR-Rezeptor,
(iii) eine markierte DNA-Sonde enthält, die eine Nukleotidsequenz enthält, an die die DNA-Bindungsdomäne eines Ligand-FXR-RXR-Komplexes spezifisch bindet;
(2) Bereitstellung einer zweiten Mischung, die
(i) einen FXR-Rezeptor,
(ii) einen mutierten RXR-Rezeptor ("RXRm"), der eine funktionelle DNA-Bindungsdomäne enthält und die eine Mutation in der Ligandenbindungsdomäne hat, die eine wesentliche Aktivierung durch RXR-Liganden verhindert, die aber ansonsten die Fähigkeit des mutierten RXR-Rezeptors zur Bildung von Heterodimeren mit FXR oder solcher Heterodimere zur Rekrutierung von Coaktivator nicht wesentlich beeinflusst,
(iii) eine markierte DNA-Sonde enthält, die eine Nukleotidsequenz enthält, an die die DNA-Bindungsdomäne des Ligand-FXR-RXR-Komplexes spezifisch bindet,
(3) in Kontakt bringen der ersten und zweiten Mischung mit der zu screenenden Verbindung,
(4) Bestimmung, ob die Verbindung die Bindung eines FXR-RXR-Heterodimers an die DNA-Sonde bewirkt und
(5) Bestimmung, ob die zu screenende Verbindung die Bindung eines FXR-RXRm-Heterodimers an die DNA-Sonde bewirkt.

5. Verfahren nach Anspruch 4, das weiterhin das in Kontakt bringen der ersten und zweiten Mischung mit einem bekannten FXR-Liganden und die Selektion von Verbindungen umfasst, die die Fähigkeit dieses bekannten FXR-Liganden hemmen, die Bindung des FXR-RXR-Heterodimers an die DNA-Sonde bewirken.

6. Verfahren nach Anspruch 5, das weiterhin die Selektion von Verbindungen umfasst, die keine wesentliche Bindung des FXR-RXRm-Heterodimers an die DNA-Sonde bewirken.

7. Verfahren zum Screenen nach zur Modulation der FXR-vermittelten Gentranskription nützlichen Verbindungen, das das in Kontakt bringen einer Mischung aus FXR, RXR und einem FXR-RXR-Coaktivator mit einer Verbindung und die Bestimmung umfasst, ob diese Verbindung die Rekrutierung von Coaktivator durch ein FXR-RXR-Heterodimer fördert, worin RXR eine RXR-Mutante (RXRm) ist, die eine funktionelle DNA-Bindungsdomäne enthält und die eine Mutation in der Liganden-Bindungsdomäne hat, die eine wesentliche Aktivierung durch RXR-Liganden verhindert, die aber ansonsten die Fähigkeit des mutierten RXR-Rezeptors zur Bildung von Heterodimeren mit FXR oder solcher Heterodimere zur Rekrutierung von Coaktivator nicht wesentlich beeinflusst.

8. Verfahren zum Screenen von Verbindungen mit FXR-Antagonist-Aktivität, die das in Kontakt bringen einer Mischung von FXR, RXR, eines FXR-RXR-Coaktivators und einem bekannten FXR-Agonisten mit einer Verbindung und die Bestimmung umfasst, ob diese Verbindung die agonist-geförderte Coaktivator-Rekrutierung durch ein FXR-RXR-Heterodimer hemmt, worin RXR eine RXR-Mutante ("RXRm") ist, die eine funktionelle DNA-Bindungsdomäne enthält und die eine Mutation in der Liganden-Bindungsdomäne hat, die eine wesentliche Aktivierung durch RXR-Liganden verhindert, die aber ansonsten die Fähigkeit des mutierten RXR-Rezeptors zur Bildung von Heterodimeren mit FXR oder solcher Heterodimere zur Rekrutierung von Coaktivator nicht wesentlich beeinflusst.

9. Verfahren nach einem der Ansprüche 1 bis 8, worin RXRm eine Asp332-Pro-Mutante des menschlichen RXRα ist.

10. Verfahren nach Anspruch 7 oder 8, worin der Coaktivator ein Polypeptid oder ein aktives Fragment davon ist, das ein Peptidmotiv enthält, das mit dem FXR-RXR-Heterodimer in ligandenabhängiger Weise interagiert.

11. Verfahren nach Anspruch 7 oder 8, worin der Coaktivator aus CBP, SRC-1 und PDB/DRIP205/TRAP220 ausgewählt ist.

12. Verfahren nach Anspruch 7 oder 8, worin der Coaktivator GRIP1 ist.

13. Verfahren nach Anspruch 4, 5 oder 6, worin die DNA-Sonde die Nukleotidsequenz von SEQ ID Nr. 3 oder SEQ ID Nr. 4 umfasst.

14. Verfahren zum Screenen nach zur Modulation der FXR-vermittelten Gentranskription nützlicher Verbindungen umfassend
(a) die Transfektion von Säugerzellen mit einem für FXR kodierenden Gen unter Kontrolle eines funktionstüchtigen Promotors;
(b) Transfektion dieser Zellen mit einem funktionstüchtigen Reportergen unter der Kontrolle eines mit einer für ein funktionstüchtiges Antwortelement kodierenden DNA-Sequenz verbundenen Promoters, an den ligandaktiviertes FXR oder ein FXR-Komplex zur Initiation der Transkription dieses Reportergens bindet,
(c) Kultivieren dieser Zellen in Gegenwart einer zu screenenden Verbindung und
(d) Überwachung dieser Zellen auf Transkription oder Expression des Reportergens als Hinweis der FXR-Aktivierung, worin diese Zellen mit einem für ein Gallensäuretransportmolekül kodierenden Gen unter Kontrolle eines funktionstüchtigen Promotors transferiert sind.

15. Verfahren nach Anspruch 14, worin diese Zellen in Gegenwart eines bekannten FXR-Liganden kultiviert werden und die Abnahme der Transkription oder Expression des Reportergens ein Hinweis darauf ist, dass die gescreente Verbindung ein FXR-Antagonist ist.

16. Verfahren nach Anspruch 15, worin das Verfahren verwendet wird, um zur Erhöhung des Cholesterinabbaus nützliche Verbindungen zu identifizieren.

## Revendications

1. Procédé de criblage de composés utiles pour la modulation de la transcription de gènes par médiation du FXR, qui comprend les étapes de : mettre en contact un mélange de FXR et de RXR avec un composé ; et de déterminer si ledit composé favorise la formation d'un hétérodimère FXR-RXR, dans lequel le RXR est un RXR mutant (RXRm), qui contient un domaine fonctionnel de liaison à l'ADN et qui a une mutation dans le domaine de liaison au ligand, ce qui empêche une activation essentielle par les ligands du RXR mais n'affecte pas autrement substantiellement la faculté du récepteur RXR mutant de former des hétérodimères avec le FXR.

2. Procédé de criblage de composés ayant une activité d'antagoniste du FXR, lequel procédé comprend les étapes de : mettre en contact un mélange de FXR et de RXR avec un composé ; et de déterminer si ledit composé inhibe la formation d'un hétérodimère FXR-RXR favorisée par un agoniste, dans lequel le RXR est un RXR mutant (RXRm) qui contient un domaine fonctionnel de liaison à l'ADN et qui a une mutation dans le domaine de liaison au ligand, ce qui-empêche une activation essentielle par les ligands du RXR mais n'affecte pas autrement substantiellement la faculté du récepteur RXR mutant de former des hétérodimères avec le FXR.

3. Procédé selon la revendication 2, dans lequel le mélange contient en outre un agoniste connu du FXR.

4. Procédé de criblage de composés ayant une activité de modulation du catabolisme du cholestérol, qui comprend les étapes de :
(1) fournir un premier mélange qui contient :
(i) un récepteur FXR,
(ii) un récepteur RXR,
(iii) une sonde d'ADN marquée, qui contient une séquence de nucléotides à laquelle se lie spécifiquement le domaine de liaison à l'ADN d'un complexe ligand-FXR-RXR ;
(2) fournir un deuxième mélange, qui contient :
(i) un récepteur FXR,
(ü) un récepteur RXR mutant ("RXRm"), qui contient un domaine fonctionnel de liaison à l'ADN et qui a une mutation dans le domaine de liaison au ligand, ce qui empêche une activation essentielle par les ligands du RXR mais n'affecte pas autrement substantiellement la faculté du récepteur du RXR mutant de former des hétérodimères avec le FXR ou celle de tels hétérodimères de recruter un co-activateur,
(iii) une sonde d'ADN marquée, qui contient une séquence de nucléotides à laquelle se lie spécifiquement le domaine de liaison à l'ADN d'un complexe ligand-FXR-RXR ;
(3) mettre en contact lesdits premier et deuxième mélanges avec le composé étant criblé ;
(4) déterminer si le composé provoque la liaison d'un hétérodimère FXR-RXR à la sonde d'ADN ; et
(5) déterminer si le composé criblé provoque la liaison d'un hétérodimère FXR-RXRm à la sonde d'ADN.

5. Procédé selon la revendication 4, qui comprend en outre les étapes de : mettre en contact les premier et deuxième mélanges avec un ligand connu du FXR ; et de choisir des composés qui inhibent la faculté dudit ligand connu du FXR de faire que l'hétérodimère FXR-RXR se lie à la sonde d'ADN.

6. Procédé selon la revendication 5, qui comprend en outre de choisir des composés qui ne provoquent pas de liaison substantielle de l'hétérodimère FXR-RXRm avec la sonde d'ADN.

7. Procédé de criblage de composés utiles pour la modulation de la transcription de gènes par médiation du FXR, qui comprend les étapes de : mettre en contact un mélange de FXR, de RXR et d'un co-activateur du FXR-RXR avec un composé ; et de déterminer si ledit composé favorise le recrutement d'un co-activateur par un hétérodimère FXR-RXR, dans lequel le RXR est un RXR mutant (RXRm) qui contient un domaine fonctionnel de liaison à l'ADN et qui a une mutation dans le domaine de liaison au ligand, ce qui empêche une activation essentielle par les ligands du RXR mais n'affecte pas autrement substantiellement la faculté du récepteur RXR mutant de former des hétérodimères avec le FXR ou celle de tels hétérodimères de recruter un co-activateur.

8. Procédé de criblage de composés ayant une activité d'antagoniste du FXR, lequel procédé comprend les étapes de : mettre en contact un mélange de FXR, de RXR, d'un co-activateur de FXR-RXR et d'un agoniste connu du FXR avec un composé ; et de déterminer si ledit composé inhibe le recrutement, favorisé par l'agoniste, d'un co-activateur par un hétérodimère FXR-RXR, dans lequel le RXR est un RXR mutant ("RXRm") qui contient un domaine fonctionnel de liaison à l'ADN et qui a une mutation dans le domaine de liaison au ligand, ce qui empêche une activation essentielle par les ligands du RXR mais n'affecte pas autrement substantiellement la faculté du récepteur RXR mutant de former des hétérodimères avec le FXR ou celle de tels hétérodimères de recruter un co-activateur.

9. Procédé selon l'une quelconque des revendications 1 à 8 dans lequel le RXRm est un mutant Asp332→Pro du RXRα humain.

10. Procédé selon les revendications 7 ou 8 dans lequel le co-activateur est un polypeptide ou un fragment actif de celui-ci qui contient un motif peptidique, lequel interagit avec l'hétérodimère FXR-RXR d'une manière dépendante du ligand.

11. Procédé selon les revendications 7 ou 8 dans lequel le co-activateur est choisi parmi les CBP, SRC-1 et PDB/DRIP205/TRAP220.

12. Procédé selon les revendications 7 ou 8 dans lequel le co-activateur est le GRIP1.

13. Procédé selon les revendications 4, 5 ou 6 dans lequel la sonde d'ADN comprend la séquence nucléotidique SEQ ID N° 3 ou SEQ ID N°4.

14. Procédé de criblage de composés utiles pour la modulation de la transcription de gènes par médiation du FXR, qui comprend les étapes de :
(a) transfecter des cellules de mammifère avec un gène codant pour un FXR, qui est sous le contrôle d'un promoteur opérationnel ;
(b) transfecter lesdites cellules avec un gène indicateur opérationnel étant sous le contrôle d'un promoteur lié à une séquence d'ADN, qui code pour un élément de réponse opérationnel auquel se lie un FXR ou un complexe FXR activé par un ligand pour débuter la transcription dudit gène indicateur ;
(c ) mettre en culture lesdites cellules en présence d'un composé criblé ; et
(d) surveiller desdites cellules pour détecter la transcription ou l'expression du gène indicateur, comme indication d'une activation du FXR, dans lequel lesdites cellules sont transfectées avec un gène codant pour une molécule transporteuse d'acide biliaire étant sous le contrôle d'un promoteur opérationnel.

15. Procédé selon la revendication 14, dans lequel lesdites cellules sont cultivées en présence d'un ligand connu du FXR et dans lequel la diminution de la transcription ou de l'expression d'un gène indicateur est une indication que le composé criblé est un antagoniste du FXR.

16. Procédé selon la revendication 15 dans laquelle le procédé est utilisé pour identifier des composés qui sont utiles pour augmenter le catabolisme du cholestérol.
